# EUROPEAN PATENT APPLICATION

(11) **EP 0 839 801 A2**
(43) Date of publication of application: **06.05.1998**
(21) Application number: 97118871.9
(22) Date of filing: 30.10.1997
(51) Int. Cl.: C07C 231/12

(54) **Production of benzophenone derivatives**

(30) Priority: 01.11.1996 JP 292118/96
(71) Applicant: TAKEDA CHEMICAL INDUSTRIES, LTD., Chuo-ku, Osaka 541 (JP)
(72) Inventor: Nakatani, Hiroshi, Amagasaki, Hyogo 661 (JP); Yamashita, Makoto, Amagasaki, Hyogo 661 (JP); Kuroda, Kazunori, Yamaguchi 743 (JP)
(74) Representative: von Kreisler, Alek, Dipl.-Chem.

(57) **Abstract**

A method for producing a compound of the formula: 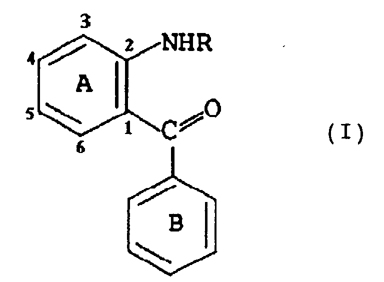 wherein R is an acyl group or a silyl group, the rings A and B respectively may have one to four substituent(s), or a salt thereof, which comprists reacting a compound of the formula: 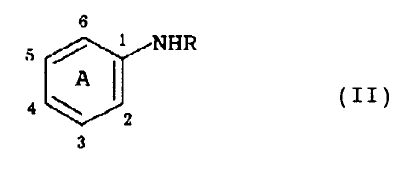 wherein R has the same meaning as defined above, the ring A may have one to four substituents at the position(s) other than 2-position, or a salt thereof, with a compound of the formula: 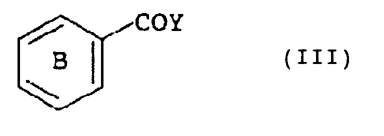 wherein Y is a hydroxyl group or a halogen atom, the ring B may have one to four substituent(s), or its salt, in the presence of a catalyst other than polyphosphonic acid.

The method produces aminobenzophenone derivative of the formula (I) in a high purity, a high yield, with a conventional and commercially advantageous procedure.

## Description

### FIELD OF THE INVENTION

The present invention relates to a method for producing a benzophenone derivative which is an important intermediate for the production of quinoline or quinazoline derivatives of value as antiinflammatory, antirheumatic, and other drugs.

### BACKGROUND OF THE INVENTION

European Patent Application Publication No. 608,870A (Japanese Patent Application Laid-open No. 7-118266) discloses a quinoline or quinazoline derivative of use as an antiinflammatory agent, particularly a drug for treating arthritis, or an antirheumatic drug. As a starting material for producing the above quinoline or quinazoline derivative, a benzophenone derivative having an amino substituent at the 2-position is disclosed.

Japanese Patent Application Laid-open No. 60-87248 discloses a process for producing an 2-aminobenzophenone compound which comprises reacting a benzoyl chloride with a para-substituted aniline compound in the presence of phosphorus pentoxide and a process for producing an 2-aminobenzophenone compound which comprises reacting a benzoic acid compound with a para-substituted aniline compound in the presence of phosphorus pentachloride and/or phosphorus oxychloride. However, this publication does not disclose any production technology using a Friedel-Crafts catalyst such as a metal halide catalyst and, moreover, the starting compound for this production process is limited to a para-substituted aniline compound having a halogen atom or a lower alkyl group as the substituent.

Japanese Patent Application Laid-open No. 6-172252 discloses a process for producing a benzophenone compound which comprises reacting a benzene derivative which may optionally have 1 to 3 substituents selected from hydroxy, alkoxy, alkyl, aryl and halogen with an aromatic carboxylic acid compound having 1 to 3 substituents selected from hydroxy, alkoxy, alkyl, aryl and halogen in the presence of a Friedel-Crafts catalyst and either a C₁₋₈ alcohol or water.

Japanese Patent Application Laid-open Nos. 4-21651, 5-70397 and 6-172253 disclose processes for producing polyhydroxybenzophenone compounds wherein a phenol compound and an aromatic carboxylic acid compound are reacted in the presence of a Friedel-Crafts catalyst using the reaction solvent selected from an alkanesulfonic acid, sulfolene, and an alkyl or arylphosphine oxide (or sulfide), respectively.

### OBJECT OF THE INVENTION

Benzophenone derivatives are not only of value as synthetic intermediates for the production of various medicines such as antiinflammatory agents, antirheumatic agents, etc. as mentioned above but also of use as synthetic intermediates for agrochemicals, dyes, polymers, ultraviolet absorbers, synthetic resin stabilizers, photoresist sensitizers, etc., and there has been a demand for establishment of a commercial process for their production.

The object of the present invention is to provide a method for producing benzophenone derivatives with a commercially advantageous procedure.

### SUMMARY OF THE INVENTION

In view of the above state of the art, the inventors of the present invention did intensive research and found that a benzophenone derivative having a substituted amino group in the 2-position can be produced in a good yield and a high purity without formation of by-products by reacting an N-phenyl-substituted amide compound with a benzoic acid compound or a benzoyl halide. The present invention has been developed on the basis of the above finding.
The present invention is:
(1) A method for producing a compound of the formula: 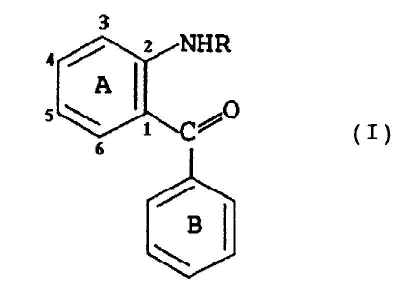 wherein R is an acyl group or a silyl group, the rings A and B respectively may have one to four substituent(s), or a salt thereof, which comprises reacting a compound of the formula: 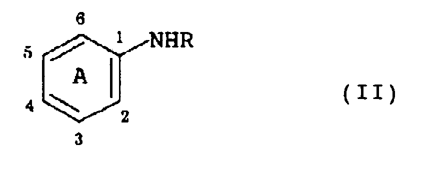 wherein R has the same meaning as defined above, the ring A may have one to four substituents at the other position(s) than the 2-position, or a salt thereof, with a compound of the formula: 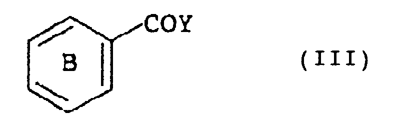 wherein Y is a hydroxyl group or a halogen atom, the ring B may have one to four substituent(s), or its salt, in the presence of a catalyst except polyphosphonic acid,
(2) The method as claimed in the item (1), wherein the ring A of the compound (I) has a substituent at the 5-position.
(3) The method as claimed in the item (1), wherein the ring A has substituents at the 4- and 5-positions,
(4) The method as claimed in the item (1), wherein the catalyst is a Friedel-Crafts catalyst,
(5) The method as claimed in the item (4), wherein the Friedel-Crafts catalyst is a halogenated metal,
(6) The method as claimed in the item (5), wherein the halogenated metal is a halogenated tin,
(7) The method as claimed in the item (4), wherein phosphorous oxychloride or phosphorous trichloride is further used as a catalyst,
(8) The method as claimed in the item (1), wherein the substituent(s) on the rings A and/or B are respectively a substituted hydroxy, an alkyl group which may optionally be substituted, an acyl group which may optionally be substituted or a halogen atom,
(9) The method as claimed in the item (1), wherein at least one substituent on the rings A or B is C₁₋₆ alkoxy group,
(10) The method as claimed in the item (1), wherein the substituents on the rings A and B are respectively two C₁₋₆ alkoxy groups,
(11) The method as claimed in the item (1), wherein R is a group of the formula: -CO-R¹, wherein R¹ is a C₁₋₆ alkyl group which may optionally be substituted,
(12) The method as claimed in the item (1), wherein R is an acetyl group.

### DETAILED DESCRIPTION OF THE INVENTION

The following are definitions of the various terms relevant to the invention as used in this specification and preferred examples for each definition.

Referring to the above formulas, rings A and B may respectively have 1 to 4 substituents (provided, however, that with regard to ring A, the 2-position, that is to say either one of the two ortho-positions, is unsubstituted). The ring B may have 1 to 4 unreactive substituents.

The substituent group that may be present on ring A and/or ring B, which is substantially unreactive, includes halogen, nitro, alkyl which may be substituted, hydroxy which may be substituted, thiol which may be substituted, amino which may be substituted, acyl which may be substituted, carboxyl which is esterified, carbamoyl, N-mono-C₁₋₄ alkylcarbamoyl, N,N-di-C₁₋₄ alkylcarbamoyl, cycloaminocarbonyl, C₁₋₆ alkanoylamide, carbamoylamino, N-C₁₋₄ alkylcarbamoylamino, N,N-di-C₁₋₄ alkylcarbamoylamino, C₁₋₁₀ alkylsulfinyl, C₁₋₁₀ alkylsulfonyl, 3- through 6-membered heterocyclic amino, cyano, sulfo, sulfino, phosphono and sulfamoyl. Particularly preferred are hydroxy which may be substituted, alkyl which may be substituted, acyl which may be substituted, and halogen.

Such halogen atoms as the substituent on the ring A or B include atoms of fluorine, chlorine, bromine and iodine, with preference given to atoms of fluorine and chlorine.

The alkyl group in the alkyl group, which may be substituted, as the substituent on the ring A or B may be any one having 1 to 10 carbon atoms, whether linear, branched (C₃₋₁₀) or cyclic (C₃₋₁₀), exemplified by methyl, ethyl, propyl, isopropyl, butyl, isobutyl, sec-butyl, tert-butyl, pentyl, isopentyl, neopentyl, hexyl, heptyl, octyl, nonyl, decyl, cyclopropyl, cyclobutyl, cyclopentyl, cyclohexyl and cycloheptyl. As the substituted C₁₋₁₀ alkyl, mention is made of C₁₋₁₀ alkyl group which is substituted with one to three halogen atom (e.g. fluorine, chlorine, bromine, iodine), amino, hydroxyl, amido, cyano, nitro, carboxyl which is esterified with C₁₋₃ alkyl (e.g. methyl, ethyl, propyl, isoprpyl). As the preferable substituted C₁₋₁₀ alkyl, mention is made of halogenated C₁₋₆ alkyl (e.g. chloromethyl, dichloromethyl, trifluoromethyl, trifluoroethyl).

The hydroxyl group, which may be substituted, as the substituent on the ring A or B is exemplified by the hydroxyl group and hydroxyl groups having an appropriate substituent, particularly a substituent used as a hydroxyl group protecting group, such as alkoxy, alkenyloxy, alkynyloxy and acyloxy. Said alkoxy is preferably an alkoxy having 1 to 10 carbon atoms (e.g., methoxy, ethoxy, propoxy, isopropoxy, butoxy, isobutoxy, sec-butoxy, tert-butoxy, pentoxy, isopentoxy, neopentoxy, hexyloxy, heptyloxy, nonyloxy, cyclobutoxy, cyclopentoxy, cyclohexyloxy). Said alkenyloxy is exemplified by alkenyloxys having 2 to 10 carbon atoms such as allyloxy, crotyloxy, 2-pentenyloxy, 3-hexenyloxy, 2-cyclopentenylmethoxy and 2-cyclohexenylmethoxy. The C₂₋₁₀ alkynyloxy is exemplified by ethynyloxy, 2-propionyloxy. Said acyloxy is preferably an alkanoyloxy having 2 to 4 carbon atoms (e.g., acetyloxy, propionyloxy, n-butyryloxy, isobutyryloxy). The preferable hydroxyl group which may be substituted for is C₁₋₁₀ alkoxy, more preferably C₁₋₆ alkoxy such as methoxy, ethoxy, propoxy and isopropoxy.

The thiol group, which may be substituted, as the substituent on the ring A or B is exemplified by the thiol group and thiol groups having an appropriate substituent, particularly a substituent used as a thiol group protecting group, such as alkylthio, alkenylthio, alkynylthio and acylthio. Said alkylthio is preferably an alkylthio having 1 to 10 carbon atoms (e.g., methylthio, ethylthio, propylthio, isopropylthio, n-butylthio, isobutylthio, sec-butylthio, tert-butylthio, pentylthio, isopentylthio, neopentylthio, hexylthio, heptylthio, nonylthio, cyclobutylthio, cyclopentylthio, cyclohexylthio). Said alkenylthio is exemplified by C₂₋₁₀ alkenylthio (e.g. allylthio, crotylthio, 2-pentenylthio, 3-hexenylthio, 2-cyclopentenylthio, 2-cyclohexenylthio). Said alkynylthio is exemplified by C₂₋₁₀ alkynylthio (e.g. ethynylthio, 2-propylthio). Said acylthio is preferably an alkanoylthio having 2 to 4 carbon atoms (e.g., acetylthio, propionylthio, n-butyrylthio, isobutyrylthio).

The amino group, which may be substituted, as the substituent on the ring A or B is exemplified by an amino group and amino groups substituted with 1 or 2 of alkyl groups having 1 to 10 carbon atoms, alkenyl groups having 2 to 10 carbon atoms, alkynyl groups having 2 to 10 carbon atoms, aromatic groups, heterocyclic groups and acyl groups having one to ten carbon atoms (e.g., methylamino, dimethylamino, ethylamino, diethylamino, dibutylamino, diallylamino, cyclohexylamino, phenylamino, N-methyl-N-phenylamino, acetylamino, propionylamino, benzoylamino, nicotinoylamino etc.).

The acyl group is exemplified by formyls and groups resulting from binding formyl with an alkyl group having 1 to 10 carbon atoms, an alkenyl group having 2 to 10 carbon atoms, an alkynyl group having 2 to 10 carbon atoms or an aromatic group and a carbonyl group (e.g., acetyl, propionyl, butyryl, isobutyryl, valeryl, isovaleryl, pivaloyl, hexanoyl, heptanoyl, octanoyl, cyclobutanoyl, cyclopentanoyl, cyclohexanoyl, cycloheptanoyl, crotonyl, 2-cyclohexenecarbonyl, benzoyl, nicotinoyl). The substituents on the acyl group are the same as those on the alkyl group.

The carboxyl group, which is esterified, as the substituent on the ring A or B is exemplified by alkyloxycarbonyl, alkenyloxycarbonyl, alkynyloxycarbonyl and acyloxycarbonyl. These groups are exemplified by a group of the formula: -COOR³, wherein R³ is a C₁₋₆ alkyl group. The alkyl group in said alkyloxycarbonyl group and so forth is exemplified by alkyl groups having 1 to 6 carbon atoms such as methyl, ethyl, propyl, isopropyl, butyl, isobutyl, sec-butyl and tert-butyl.

The N-mono-C₁₋₄ alkylcarbamoyl as a substituent on ring A or ring B includes but is not limited to N-methylcarbamoyl, N-ethylcarbamoyl, N-propylcarbamoyl, N-isopropylcarbamoyl, and N-butylcarbamoyl.

The N,N-di-C₁₋₄ alkylcarbamoyl as a substituent on ring A or ring B includes but is not limited to N,N-dimethylcarbamoyl, N,N-diethylcarbamoyl, N,N-dipropylcarbamoyl, and N,N-dibutylcarbamoyl.

The cycloaminocarbonyl as a substituent on ring A or ring B includes but is not limited to 1-aziridinylcarbonyl, 1-azetidinylcarbonyl, 1-pyrrolidinylcarbonyl, 1-piperidinylcarbonyl, and N-methylpiperazinylcarbonyl, morpholinocarbonyl and thiomorpholinocarbonyl.

The C₁₋₆ alkanoylamido as a substituent on ring A or ring B includes but is not limited to formamido, acetamido, trifluoroacetamido, propionamido, butyrylamido, and isobutyrylamido.

The N-C₁₋₄ alkylcarbamoylamino as a substituent on ring A or ring B includes but is not limited to N-methylcarbamoylamino, N-ethylcarbamoylamino, N-propylcarbamoylamino, N-isopropylcarbamoylamino, and N-butylcarbamoylamino.

The N,N-di-C₁₋₄ alkylcarbamoylamino as a substituent on ring A or ring B includes but is not limited to N,N-dimethylcarbamoylamino, N,N-diethylcarbamoylamino, N,N-dipropylcarbamoylamino, and N,N-dibutylcarbamoylamino.

The C₁₋₁₀ alkylsulfinyl as a substituent on ring A or ring B includes but is not limited to methylsulfinyl, ethylsulfinyl, propylsulfinyl, and butylsulfinyl. The C₁₋₁₀ alkylsulfonyl includes but is no limited to methylsulfonyl, ethylsulfonyl, propylsulfonyl, and butylsulfonyl.

The 3- through 6-membered heterocyclic amino groups includes 3- through 6-membered heterocyclic amino groups optionally containing 1 to 3 hetero-atoms selected from oxygen, sulfur and nitrogen in addition to carbon atoms and one nitrogen atom as ring members (e.g. aziridinyl, azetidinyl, pyrrolidinyl, pyrrolinyl, pyrrolyl, imidazolyl, pyrazolyl, imidazolidinyl, piperidino, morpholino, thiomorpholino, dihydropyridyl, pyridyl, N-methylpiperazinyl, N-ethylpiperazinyl, etc.).

The substituents mentioned above for ring A may be present in any positions other than the 2-position (i.e. ortho-position) of the ring. The substituents for ring B may be present in any positions of the ring. Preferably, ring A is substituted at the 5-position of the compound (I) (4-position of the compound (II)), more preferably, ring A is substituted at the 4-position and/or 5-position. Preferably, ring B is substituted at the 3-position and/or 4-position. These substituents, when there are two or more, may be similar or dissimilar and the number of substitutions per ring is 1 to 4, and preferably 1 to 2.

When any of the two substituents on ring A or ring B are adjacent to each other, they may jointly form a ring of the formula -(CH₂)ₘ or -O-(CH₂)ₙ-O-, wherein m represents an integer of 3 to 5 and n represents an integer of 1 to 3, which ring includes 5- through 7-membered ring systems each formed in combination with two carbon atoms of the benzene ring.

Preferable substituent(s) on the rings A and/or B is/are respectively a substituted hydroxy, an alkyl group which may optionally be substituted, an aryl group which may optionally be substituted or a halogen atom.

The preferred mode in which the ring A is substituted, taking case (1) in which the ring A is substituted with alkoxy or hydroxy as an example, case (2) in which ring A is di-substituted with the same or different alkoxy groups (particularly at the 4- and 5-positions) and case (3) in which the ring A is substituted with methylenedioxy at the 4,5-positions. Particularly preferred is the case in which the ring A is di-substituted with C₁₋₆ alkoxy group, preferably methoxy, preferably at the 4- and 5-positions.

The preferred mode in which the ring B is substituted, taking case (1) in which the ring B is substituted by alkoxy or hydroxy as an example, case (2) in which ring B is di-substituted with the same or different alkoxy groups (particularly methoxy and/or isopropoxy, most preferably at the 3- and 4-positions) and case (3) in which the ring B is substituted with methylenedioxy. Particularly preferred is the case in which ring B is di-substituted with C₁₋₆ alkoxy, preferably methoxy, preferably at the 3- and 4-positions.

Referring to formula (II) and formula (I), R represents acyl or silyl. The acyl mentioned just above can be represented by the formula -CO-R¹ (R¹ represents group which may optionaly be substituted with a C₁₋₅ alkyl group). The C₁₋₆ alkyl for R¹ includes methyl, ethyl, propyl, isopropyl, butyl, isobutyl, sec-butyl, tert-butyl, pentyl, isopentyl, neopentyl, tert-pentyl, 1-ethylpropyl, etc. Particularly preferred is C₁₋₅ alkyl, more preferably C₁₋₃ alkyl such as methyl or ethyl. As the substituents on the alkyl group, mention is made of one to three halogen atom (e.g. fluorine, chlorine, bromine, iodine), amino, hydroxyl, amido, cyano, nitro, carboxyl which is esterified with C₁₋₃ alkyl (e.g. methyl, ethyl, propyl, isopropyl).

The silyl mentioned above includes silyl group which may have two or three groups selected from C₁₋₆ alkyl groups (e.g. methyl, ethyl, propyl, isopropyl, butyl, isobutyl, sec-butyl, tert-butyl, theryl) C₁₋₃ alkoxy groups (e.g. methoxy, ethoxy) and phenyl group, such as trimethylsilyl, dimethylisopropylsilyl, tert-butyl-dimethylsilyl, di-tert-butylmethylsilyl, thexyldimethylsilyl, triethylsilyl, triisopropylsilyl, triphenylsilyl, diethylisopropylsilyl, diphenylmethylsilyl, tert-butyl-diphenylsilyl, tert-butylmethoxyphenylsilyl, etc. Particularly preferred are trimethylsilyl, dimethylisopropylsilyl, tert-butyldimethylsilyl, di-tert-butylmethylsilyl, and thexyldimethylsilyl. Preferably, R in formulas (II) and (I) represents C₁₋₆ acyl such as acetyl, propionyl, butyryl, etc. More preferably, R is acetyl.

In formula (III), Y represents hydroxy or halogen, preferably hydroxy. The halogen for Y includes fluorine, chlorine, bromine, and iodine, and preferably is chlorine.

Among species of the compound (III), the species wherein Y is halogen (i.e. the compound is acid halide) can be produced by treating the corresponding compound wherein Y is hydroxy, that is to say an aromatic carboxylic acid, in the per se known manner, for example using an inorganic acid halide (e.g. phosphorus pentachloride, phosphorus trichloride, phosphorus tribromide, thionyl chloride, thionyl bromide, phosphoryl chloride, oxalyl chloride, etc.; preferably thionyl chloride or phosphoryl chloride).

When the compound (I), (II) and/or (III) contains a basic group such as amino group, they may form a salt with an acid such as an inorganic acid (e.g. hydrochloric acid, phosphoric acid, hydrobromic acid, sulfuric acid), organic acid (e.g. acetic acid, formic acid, propionic acid, fumaric acid, maleic acid, succinic acid, citric acid, malic acid, oxalic acid, benzoic acid, methanesulfonic acid, bensenesulfonic acid.

When the compound (I), (II) and/or (III) contains an acidic group such as thiol gorup, they may form a salt with an inorganic base such as an alkaline metal or an alkaline earth metal (e.g. sodium, potassium calcium, magnesium), or ammonia, or with an organic base such as tri-C₁₋₃ alkylamine (e.g. triethylamine).

Among species of the compound of formula (II), the preferred are
N-(3,4-dimethoxyphenyl)acetamide,
N-(3-hydroxy-4-methoxyphenyl)acetamide,
N-(4-hydroxy-3-methoxyphenyl)acetamide, and
N-(3,4-methylenedioxyphenyl)acetamide.
The most preferred is N-(3,4-dimethoxyphenyl)acetamide.

Among species of the compound of formula (III), the particularly preferred are
3,4-dimethoxybenzoic acid,
3-hydroxy-4-methoxybenzoic acid,
4-hydroxy-3-methoxybenzoic acid, and
3,4-methylenedioxybenzoic acid.
The most preferred is 3,4-dimethoxybenzoic acid.

Among species of the compound of formula (I), the particularly preferred are
2-acetylamino-3',4,4',5-tetramethoxybenzophenone,
2-acetylamino-3'-isopropoxy-4,4',5-trimethoxybenzophenone,
2-acetylamino-4-hydroxy-3',4',5-trimethoxybenzophenone,
2-acetylamino-3'-hydroxy-4,4',5-trimethoxybenzophenone, and
2-acetylamino-5-hydroxy-3',4,4'-trimethoxybenzophenone.
The most preferred is 2-acetylamino-3',4,4',5-tetramethoxybenzophenone.

The organic solvent for use in the reaction according to the process of the invention can be judiciously selected from among the solvents which are inert to the compound of formula (II) or its salt, compound of formula (III) or its salt, and aminobenzophenone compound of formula (I) or its salt and do not interfere with the reaction. Preferred are halogenated hydrocarbons such as dichloromethane, 1,2-dichloroethane, 1,1-dichloropropane, 1,2-dichloropropane, 1,3-dichloropropane₁ 2,2-dichloropropane, 1,4-dichlorobutane, 2,3-dichlorobutane, 1-chlorobutane, 2-chlorobutane, chloroform, etc. The proportion of the organic solvent is generally at least about 5 weight equivalents, preferably about 10 to about 15 weight equivalents based on the compound of formula (II) or its salt.

The relative amount of the compound of formula (II) or its salt and compound of formula (III) or its salt for use as starting materials in the process of the invention is generally about 1 to about 3 moles and preferably about 1.1 to about 1.5 moles of compound (III) or its salt per mole of compound (II) or its salt.

The present reaction is conducted in the presence of a catalyst other than polyphosphoric acid.

The reaction according to the present invention is preferably conducted in the presence of a Friedel-Crafts catalyst. The Friedel-Crafts catalyst that can be used includes metal halide catalysts (e.g. aluminum halide, gallium halide, zirconium halide, antimony halide, molybdenum halide, zinc halide, tin halide, iron halide, boron halide, titanium halide, bismuth halide, etc.). Particularly preferred are tin halides such as tin(II) chloride, tin(IV) chloride, tin(II) bromide, and tin(IV) bromide. The proportion of the Friedel-Crafts catalyst to be used is about 1 to about 3 moles, preferably about 1.1 to about 1.8 moles, per mole of compound (II) or its salt.

Furthermore, the reaction according to the process of the invention is preferably carried out using a phosphorus halide (e.g. phosphorus oxychloride, phosphorus trichloride) in combination with said Friedel-Crafts catalyst. As a specific example, the combination of tin chloride and phosphorus oxychloride can be mentioned. The phosphorus halide is used in a proportion of about 3 to about 12 moles, preferably about 5 to about 8.5 moles, per mole of compound of formula (II) or its salt.

The reaction temperature for carrying out the process of the invention is about 0° to about 83°C, preferably about 40° to 50°C. When the reaction temperature is low, the reaction rate tends to be decreased.

The reaction time for the process of the invention is about 5 to about 30 hours, preferably about 12 to about 16 hours. The reaction time should be adjusted according to the reaction temperature and the kind and amount of the catalyst.

The aminobenzophenone derivative (I) or its salt produced by the process of the invention can be isolated by the per se known separation-purification procedures such as concentration, concentration under reduced pressure, solvent extraction, and crystallization. If desired, the compound can be further purified by a per se known purification technique such as recrystallization.

The compound of formula (I) that has been precipitated by a per se known procedure, such as solvent extraction, concentration, or concentration under reduced pressure, at the end of the reaction can be subjected to the next deacylation or other reaction in a one-pot system, i.e. without prior isolation, and/or converted to a salt (e.g. hydrochloride) of compound (I). Then, by or in accordance with the methods described in European Patent Application Publication No. 608870A (Japanese Patent Application Laid-open No. 7-118266, PCT International Publication NO. WO96/00223 (Japanese Patent Application Laid-open No. 8-67679), the compound mentioned below is produced. For instance, the product compound can be derivatized to a variety of medicinally useful end-product compounds such as antiinflammatory agents and/or antirheumatic agents. Specifically, the methods described in the following reference examples, among other methods, or analogous methods thereto can be employed for the purpose.

The present method produces aminobenzophenone derivative of the formula (I) in a high purity, in a high yield, with a convenient and commercially advantageous procedure.

### EXAMPLES

The present invention is explained more detailedly in the following working examples, but is not limited to those illustrated in the Examples. In the following, percent "%" denotes weight percent unless otherwise noted.

### Reference Example 1.

### Production of N-(3,4-dimethoxyphenyl)acetamide:

3,4-Dimethoxyaniline(153.2 g, 1 mol) was suspended in water(350 ml), and the suspension was heated to 80±5°C. With stirring the mixture at the same temperature, were dropwisely added an aqueous solution of sodium hydroxide(52.0 g/300 ml) which is 1/5 of the volume of the 3,4-dimethoxyaniline suspension and acetic anhydride(132.7 g, 1.3 mol) which is 1/5 of the volume of the 3,4-dimethoxyaniline suspension. To the resultant mixture were added in the 1/5 volume of each of the remaining portion of the aqueous solution of sodium hydroxide and the acetic anhydride at 80±5°C. After completion of the dropwise addition, the resultant mixture was allowed to react at 80±5°C for 20 minutes and left standing to be cooled to the room temperature. The mixture was further cooled to below 5°C and kept at the same temperature for 1 hour to complete the reaction. The precipitated crystals were collected by filtration with glass-filter and washed twice with water(306 ml). The precipitated wet crystals were dried at about 45°C under reduced pressure to give N-(3,4-dimethoxyphenyl)acetamide. Yield:183.43 g (93%), Purity:99%.

### Reference Example 2.

### Production of 3,4-dimethoxybenzoyl chloride:

To 3,4-dimethoxybonzoic acid(2.73 g, 15 mmol) was added thionylchloride(22.34 g, 187.8 mmol). The mixture was stirred under heating and refluxing for 1 hour. After the completion of the reaction, excessive thionylchloride was evaporated off under reduced pressure to give quantitatively 3,4-dimethoxybenzoylchloride as white yellow crystals.

### Example 1.

### Production of 2-acetylamino-3',4,4',5-tetramethoxybenzophenone:

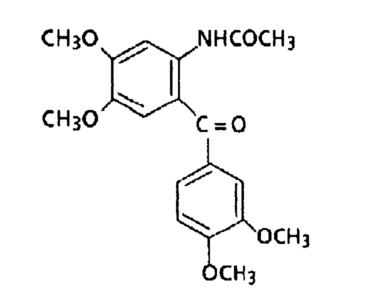

N-(3,4-dimethoxyphenyl)acetamide(9.76 g, 50 mmol) and 3,4-dimethoxybenzoic acid(13.66 g, 75 mmol) were suspended in dichloromethane(100 ml). To the resultant mixture was dropwisely added phosphoryloxychloride (65.17 g, 425 mmol), followed by stannous tetrachloride (23.45 g, 90 mmol) below 35°C. The resultant mixture was stirred under heating and refluxing for 18 hours. After completion of the reaction, dichloromethane(50 ml) was added to the resultant reaction mixture. The resultant mixture was cooled to about 10°C, followed by dropwisely adding water(250 ml) gradually to maintain the temperature below about 35°C. The resultant mixture was then stirred at room temperature for 10 minutes, left standing for 10 minutes and separated by decantation. The separated dichloromethane layer was washed with 10% aqueous NaOH(100 ml) solution to remove residual 3,4-dimethoxybenzoic acid. The resultant dichloroethane layer was again washed with pure water(100 ml), and the dichloromethane was evaporated off under atmospheric pressure to give a residue(27.5 g). To the residue was added methanol(100 ml), followed by azeotropic distillation to evaporate off the residual dichloromethane. This treatment was repeated again. To the resultant concentrated residue was added methanol(100 ml). The precipitated crystals were cooled to 10°C and left standing for 30 minute. The resultant crystals were collected by filtration with glass-filter, followed by washing twice with methanol(30 ml) through pouring. The resultant wet crystals were dried at about 40°C under reduced pressure to give 2-acetylamino-3',4,4',5-tetramethoxybenzophenone. Yield:16.4 g (88.5%),
Purity:96.8%.
H-NMR(DMSO-d₆, 90MHz) δ:2.21(3H, s, -NHCOCH₃), 3.76(3H, s, OMe), 3.93(3H, s, OMe), 3.98(3H, s, OMe), 4.00(3H, s, OMe), 6.87-7.35(4H, m, arom), 8.39(1H, s, arom), 11.05(1H, brs, -NHCOCH₃).

| Elemental analysis(C₁₉H₂₁O₆N) | | | | |
|---|---|---|---|---|
| Calculated(%): | C:63.50, | H:5.89, | O:26.71, | N:3.90 |
| Found(%) : | C:63.32, | H:5.84, | O:26.99, | N:3.85 |

Melting point: 153-156°C

### Example 2.

### Production of 2-acetylamino-3',4,4',5-tetramethoxybenzophenone:

N-(3,4-dimethoxyphenyl)acetamide(1.95 g, 10 mmol) and 3,4-dimethoxybenzoyl chloride(2.61 g, 13 mmol) were suspended in dichloromethane(20 ml), followed by dropwise addition of phosphoryloxychloride(4.60 g, 30 mmol). To the mixture was added stannous tetrachloride(4.69 g, 18 mmol) below 35°C, followed by stirring the mixture under heating and refluxing for 10 hours. After completion of the reaction, dichloromethane(10 ml) was added to the resultant mixture. The resultant mixture was cooled to about 10°C, followed by addition of water(50 ml). After the addition, the resultant mixture was stirred at room temperature for 10 minutes, left standing for 10 minutes and separated by decantation. The resultant dichloromethane layer was washed with 10% aqueous NaOH solution(20 ml) to remove residual 3,4-dimethoxybenzoic acid and again washed with pure water(20 ml). The dichloromethane was evaporated off under reduced pressure to give residue(3.85 g). To the resultant residue was added methanol(20 ml), and the mixture was subjected to azeotropic distillation under reduced pressure to remove residual dichloromethane. This treatment was repeated again. To the resultant concentrated residue was added methanol(20 ml). The precipitated crystals were cooled to 10°C and left standing for 1 hour. The resultant wet crystals were dried at about 40°C under reduced pressure to give 2-acetylamino-3',4,4',5-tetramethoxybenzophenone Yield:2.87 g(80.1%), Purity:98.4%.

### Reference Example 3.

### Production of 2-amino-3',4,4', 5-tetramethoxybenzophenone hydrochloride:

N-(3,4-dimethoxyphenyl)acetamide(19.52 g, 100mmol) and 3,4-dimethoxybenzoic acid(27.32 g, 150 mmol) were suspended in dichloromethane(200 ml), followed by dropwise addition of phosphoryloxychloride(130.34 g, 850 mmol) below 35°C. To the mixture was dropwisely added stannous tetrachloride(46.9 g, 180 mmol) below 35°C, followed by stirring the reaction mixture under heating and refluxing for 20 hours. After completion of the reaction, dichloromethane(100 ml) was added to the resultant mixture and cooled to below 10°C, followed by dropwise addition of water(500 ml) while careful attention was paid to initial heating and maintaining the temperature below about 35°C.

The resultant mixture was stirred at room temperature for 10 minutes, left standing for 10 minutes and separated by decantation. The resultant dichloromethane layer was washed with 10% aqueous NaOH solution(200 ml) to remove the residual 3,4-dimethoxybenzoic acid. The dichloromethane layer was again washed with pure water(200 ml) and distilled under atmospheric pressure to evaporate off dichloromethane to give a residue(78.4 g) including 2-acetylamino-3',4,4',5-tetramethoxybenzophenone.

To the resultant residue was added conc. hydrochloric acid(115.5 ml) and isobutanol(387 ml), followed by refluxing the mixture at 75±5°C for 2 hours under stirring. After completion of the reaction, the resultant reaction mixture was cooled to 10±5°C and left standing for 2 hours. The precipitated crystals were collected by filtration with glass-filter and washed with isobutanol(116 ml). The resultant wet crystals were dried under reduced pressure at about 45°C to give 2-amino-3',4,4',5-tetramethoxybenzophenone hydrochloride. Yield:32.2 g(89.8%), Purity:98.6%.
¹H-NMR(DMSO-d₆, 90MHz) δ:3.64(3H, s, OMe), 3.81(3H, s, OMe), 3.83(3H, s, OMe), 3.86(3H, s, OMe), 6.92-7.32(5H, m, arom), 7.72(3H, brs, -NH₂ and HCl).
Elementary analysis(C₁₇N₂₀O₅NCl)
   Calculated(%):C:57.71, H:5.70, 0:22.61, N:3.96, Cl:10.02
   Found(%) :C:57.43, H:5.71, O:22.80, N:4.36, Cl:9.70
Melting point: 198-201°C

### Reference Example 4.

### Production of 2-amino-3',4,4', 5-tetramethoxybenzophenone hydrochloride:

N-(3,4-dimethoxyphenyl)acetamide(9.76 g, 50 mmol) and 3,4-dimethoxybenzoic acid(10.02 g, 55 mmol) were suspended in dichloromethane(100 ml), followed by dropwise addition of phosphoryloxychloride(38.33 g, 250 mmol) below 35°C. To the mixture was dropwisely added stannous tetrachloride(23.45 g, 90 mmol) below 40°C, followed by stirring the reaction mixture under heating and refluxing for 24 hours. After completion of the reaction, dichloromethane(50 ml) was added to the resultant mixture and cooled to below 10°C, followed by dropwise addition of water(250 ml) while careful attention was paid to initial heating and maintaining the temperature below about 35°C.

The resultant mixture was stirred at room temperature for 10 minutes, left standing for 10 minutes and separated by decantation. The resultant dichloromethane layer was washed with 3% aqueous NaOH solution(100 ml) to remove the residual 3,4-dimethoxybenzoic acid. The dichloromethane layer was again washed with pure water(100 ml) and distilled under atmospheric pressure to evaporate off dichloromethane to give a residue(41.8 g) including 2-acetylamino-3',4,4',5-tetramethoxybenzophenone.

To the resultant residue was added conc. hydrochloric acid(57.7 ml) and isobutanol(193 ml), followed by refluxing the mixture at 75±5°C for 2 hours under stirring. After completion of the reaction, the resultant reaction mixture was cooled to 10±5°C and kept at the same temperature for 2 hours. The precipitated crystals were collected by filtration with glass-filter and washed with isobutanol(58 ml). The resultant wet crystals were dried under reduced pressure at about 45°C to give 2-amino-3',4,4',5-tetramethoxybenzophenone hydrochloride. Yield:16.1 g (89.2%), Purity:98.0%.
¹H-NMR(DMSO-d₆, 90MHz) δ:3.64(3H, s, OMe), 3.81(3H, s, OMe), 3.83(3H, s, OMe), 3.86(3H, s, OMe), 6.92-7.32(5H, m, arom), 7.72(3H, brs, -NH₂ and HCl).
Elementary analysis(C₁₇N₂₀O₅NCl)
   Calculated(%):C:57.71, H:5.70, O:22.61, N:3.96, Cl:10.02
   Found(%) :C:57.43, H:5.71, O:22.80, N:4.36, Cl:9.70
Melting point: 198-201°C

### Reference Example 5.

### Production of N-(3,4-dimethoxyphenyl)acetamide:

3,4-Dimethoxyaniline(100 g) was suspended in water(800 ml). To the suspension were added aqueous NaOH solution(34.0 g/200 ml) and acetic anhydride(86.6 g) in the volume of 1/5 at a temperature below 65°C, followed by stirring the mixture at 55 to 60°C for about 20 minutes. The reaction mixture was cooled to about 5°C and stirred for 1 hour. The precipitated crystals were collected by filtration and dried under reduced pressure to give N-(3,4-dimethoxyphenyl)acetamide as dark brown crystals. Yield:119 g (93.2.%).

A sample for measurement of IR and ¹H-NMR was recrystallized from ethyl acetate.
IR(cm⁻¹,KBr):3276, 1653, 1606, 1575, 1520, 1462
¹H-NMR(CDCl₃, 300MHz) δ:2.15(3H,s), 3.84(6H,s), 6.78(1H,d, J=8.6Hz), 6.88(1H, dd, J=8.6, 2.4Hz), 7.30(1H,d, J=2.4Hz), 7.55(1H, brs).

### Reference Example 6.

### Production of 2-acetyl-amino-3,4,4',5-tetramethoxybenzophenone:

A mixture of N-(3,4-dimethoxyphenyl)acetamide(114 g), 3,4-dimethoxybenzoic acid(117 g) and polyphosphoric acid(1203 g) was stirred at 95 to 110°C for 3 hours. To the reaction mixture was added cold water(3 L), followed by extraction with ethyl acetate(3 L). The extract was washed with aqueous 2N NaOH solution(3 L) and then with water. After concentration under reduced pressure, to the residue was added n-hexane (1.2 L) and the mixture was stirred for 30 minutes. The precipitated crystals were collected by filtration and dried under reduced pressure to give 2-acetylaminmo-3',4,4',5-tetramethoxybenzophenone as yellow crystals. Yield:129 g (61.3%).

A sample for measurement of IR and ¹H-NMR was recrystallized from ethyl acetate.
IR(cm⁻¹,KBr):2947, 1695, 1678, 1616, 1595.
¹H-NMR(CDCl₃, 300MHz) δ:2.22(3H,s), 3.76(3H,s), 3.94(3H,s), 3.98(3H,s), 4.00(3H,s), 6.93(1H,d, J=7.7Hz), 7.09(1H, s), 7.28(1H, dd, J=7.7, 1.9Hz), 7.32(1H, s), 8.38(1H, s), 11.0(1H,brs).

### Reference Example 7.

### Production of 2-amino-3',4,4',5-tetramethoxybenzophenone hydrochloride:

Concentrated hydrochloric acid(446 ml) was added to the solution of 2-acetylamino-3',4,4',5-tetramethoxybenzophenone(129 g) and isobutanol(1.49L). The mixture was refluxed for 2 hours under heating. The resultant mixture was cooled and stirred below about 5°C for 2 hours. The precipitated crystals were collected by filtration and dried under reduced pressure to give 2-amino-3',4,4',5-tetramethoxybenzophenone hydrochloride as white yellow crystals. Yield:117 g (92.3%).

A sample for measurement of IR and ¹H-NMR was recrystallized from EtOH.
IR(cm⁻¹,KBr):3618, 2839, 2567, 1657, 1628, 1583.
¹H-NMR(DMSO-d₆, 300MHz) δ:3.58(3H,s), 3.80(6H,s), 3.85(3H,s), 6.61(1H,brs), 6.93(1H,s), 7.06(1H,d, J=8.6Hz), 7.20-7.22(2H, m).

### Reference Example 8.

### Production of 2-chloromethyl-4-(3,4-dimethoxyphenyl)-6,7-dimethoxyquinoline-3-carboxylic acid ethyl ester:

2-Amino-3',4,4',5-tetramethoxybenzophenone hydrochloride(36.0 g) and 4-chloroacetoacetic acid ethyl ester(21.4 g) were refluxed in ethanol(350 ml) under stirring and heating for 7 hours. After completion of the reaction, triethylamine(10.6 g) was dropwisely added to the mixture below 20°C, followed by stirring the mixture at 5°C for 1 hour. The precipitated crystals were collected by filtration, washed twice with ethanol(50 ml) and dried under reduced pressure to give 2-chloromethyl-4-(3,4-deimethoxyphenyl)-6,7-dimethoxyquinoline-3-carboxylic acid ethyl ester. Yield:41.0 g (92%).

### Reference Example 9.

### Production of 4-amino-1-[4-(3,4-dimethoxyphenyl)-3-ethoxycarbonyl-6,7-dimethoxyquinolin-2-ylmethyl]-4H-1, 2,4-triazolium bromide:

2-Chloromethyl-4-(3,4-dimethoxyphenyl)-6,7-dimethoxyquinoline-3-carboxylic acid ethyl ester(22.5 g, 50.0 mmol, purity:99.1%), sodium bromide(5.81 g, 56.5 mmol) and 4-amino-1,2,4-triazole (5.47 g, 65.1 mmol) were suspended in dimethylformamide (DMF) (50 ml), and the suspension was stirred at 65°C for 3 hours. To the reaction mixture was added ethyl acetate(100 ml), and the precipitated crystals were collected and dried to give 4-amino-1-[4-(3,4-dimethoxyphenyl)-3-ethoxycarbonyl-6,7-dimethoxyquinolin-2-ylmethyl]-4H-1,2,4-triazolium bromide as white crystals. Yield:31.1 g (90.6%). Purity:83.6%.

A sample compound which was used for identification of a structure was purified by silica gel column chromatography(mobile phase:CH₂Cl₂:MeOH=5:1) and recrystallization(4.8% aqueous ethanol).
IR(cm⁻¹,KBr):3196, 1706, 1518, 1472.
¹H-NMR(DMSO-d₆, 90MHz) δ:0.92(3H,t, J=6.9Hz,CO₂CH₂CH₃), 3.72(3H, s, OMe), 3.77(3H, s, OMe), 3.86(3H, s, OMe), 3.96(3H, s, OMe), 3.72-4.09(2H, m, CO₂CH₂), 5.94(2H,s, CH₂N), 6.93-7.31(7H, m), 9.28(1H, s, CH=N), 10.41(1H, s, CH=N).

| Elementary analysis (C₂₅H₂₈N₅O₅Br(0.73H₂O)) | | | | |
|---|---|---|---|---|
| Calculated(%): | C:51.10, | H:5.05, | N:11.92, | Br:13.60 |
| Found(%) : | C:51.10, | H:4.91, | N:11.88, | Br:13.55 |

m. p. 183.8-184.4°C

### Reference Example 10.

### Production of 4-(3,4-dimethoxyphenyl)-6,7-dimethoxy-2-(1,2,4-triazol-1-ylmethyl)quinoline-3-carboxylic acid ethyl ester:

4-Amino-1-[4-(3,4-dimethoxyphenyl)-3-ethoxycarbonyl-6,7-dimethoxyquinolin-2-ylmethyl]-4H-1, 2,4-triazolium bromide(10.31 g, 15.0 mmol, purity:83.6%) was suspended in water(37.5 ml), followed by addition of concentrated hydrochloric acid(3.8 ml, 45 mmol) and 5.6 M aqueous NaNO₂ solution(4.00 ml, 22.5 mmol) under ice-cooling. The mixture was stirred at room temperature for 2 hours. To the reaction mixture was added 5N aqueous NaOH(8.7 ml) to neutralize it. The precipitated crystals were collected by filtration to give 4-(3,4-dimethoxyphenyl)-6,7-dimethoxy-2-(1,2,4-triazol-1-ylmethyl)quinoline-3-carboxylic acid ethyl ester as white crystals[yield:6.66 g(92.8%).
IR(cm⁻¹,KBr):1720, 1504, 1468, 1430
¹H-NMR(CDCl₃, 90MHz) δ:0.89(3H,t, J=7.1Hz,CO₂CH₂CH₃), 3.79(3H, s, OMe), 3.86(3H, s, OMe), 3.96(3H, s, OMe), 4.04(3H,s,OMe), 3.86-4.13(2H, q, J=7.1Hz,CO₂CH₂), 5.72(2H, s, CH₂N), 6.86-6.95(4H, m), 7.41(1H,s), 7.93(1H, s), 8.23(1H, s).
m. p. 175.4-176.0 °C

### Reference Example 11.

### Production of 4-(3,4-dimethoxyphenyl)-6,7-dimethoxy-2-(1,2,4-triazol-1-ylmethyl)quinoline-3-carboxylic acid ethyl ester:

4-Amino-1-[4-(3,4-dimethoxyphenyl)-3-ethoxycarbony1-6,7-dimethoxyquinolin-2-ylmethyl]-4H-1, 2,4-triazolium bromide(503 g, 0.709 mol, Purity:80.9%) was suspended in water(5.44 L), followed by addition of concentrated hydrochloric acid(159 g, 1.56 mol)and 0.63M aqueous NaNO₂(1.46 L, 0.920 mol) under ice-cooling. The resultant mixture was stirred at room temperature for 3 hours. To the reaction mixture was added 5N aqueous NaOH solution(295 ml) to neutralize it. The precipitated crystals were collected by filtration and dried washed with water to give 4-(3,4-dimethoxyphenyl)-6,7-dimethoxy-2-(1,2,4-triazol-1-ylmethyl)quinoline-3-carboxylic acid ethyl ester as white crystals. Yield:329 g (97.0%).

## Claims

1. A method for producing a compound of the formula: 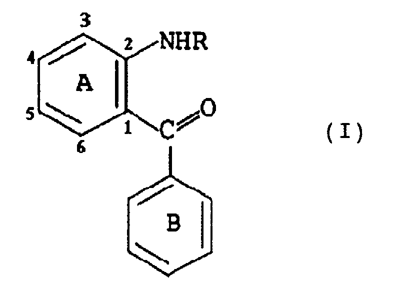 wherein R is an acyl group or a silyl group, the rings A and B respectively may have one to 4 substituent(s), or a salt thereof, which comprises reacting a compound of the formula: 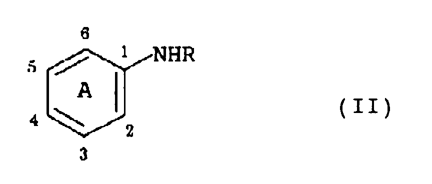 wherein R has the same meaning as defined above, the ring A may have one to four substituents at the other position(s) than the 2-position, or a salt thereof, with a compound of the formula: 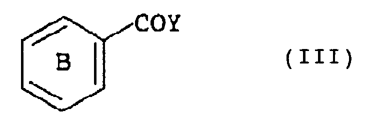 wherein Y is a hydroxyl group or a halogen atom, the ring B may have one to four substituent(s), or its salt, in the presence of a catalyst except polyphosphonic acid.

2. The method as claimed in Claim 1, wherein the ring A of the compound (I) has a substituent at 5-position.

3. The method as claimed in Claim 1, wherein the ring A of the compound (I) has substituents at the 4- and 5-positions.

4. The method as claimed in Claim 1, wherein the catalyst is a Friedel-Crafts catalyst.

5. The method as claimed in Claim 4, wherein the Friedel-Crafts catalyst is a halogenated metal.

6. The method as claimed in Claim 5, wherein the halogenated metal is a halogenated tin.

7. The method as claimed in Claim 4, wherein phosphorous oxychloride or phosphorous trichloride is further used as a catalyst.

8. The method as claimed in Claim 1, wherein the substituent(s) on the rings A and/or B are respectively a substituted hydroxy, an alkyl group which may optionally be substituted, an acyl group which may optionally be substituted or a halogen atom.

9. The method as claimed in Claim 1, wherein at least one substituent on the rings A or B is a C₁₋₆ alkoxy group.

10. The method as claimed in Claim 1, wherein the substituents on the rings A and B are respectively two C₁₋₆ alkoxy groups.

11. The method as claimed in Claim 1, wherein R is a group of the formula: -CO-R¹, wherein R¹ is a C₁₋₆ alkyl group which may optionally be substituted.

12. The method as claimed in Claim 1, wherein R is an acetyl group.
